# EUROPEAN PATENT APPLICATION

(11) **EP 4 834 424 A1**
(43) Date of publication of application: **04.11.2026**
(21) Application number: 25173881.1
(22) Date of filing: 01.05.2025
(51) Int. Cl.: A61N 5/06

(54) **TIME-OF-FLIGHT (TOF) LIGHT SENSOR OPTIMIZATION IN PHOTOBIOMODULATION (PBM)**

(71) Applicant: Sunled Life Science B.V., 1098XG Amsterdam (NL)
(72) Inventor: VAN CASTEREN, Dolf Henricus Jozef, 1098 XG Amsterdam (NL)
(74) Representative: Hoyng Rokh Monegier B.V.

(57) **Abstract**

An electro-optical arrangement (1) having a first radiation source (7) and a second radiation source (2), a receptor (3) operable to detect a reflected portion of radiation from a target and to determine a distance to the target, and a radiation control unit (30) operable to adjust at least one emission parameter of the first pulsed radiation beam based on the determined distance. Radiation beams from the first and second radiation sources are synchronized.

## Description

### TECHNICAL FIELD

The invention relates to a device that delivers radiation sufficient to induce a photobiomodulation (PBM) effect.

### BACKGROUND ART

### Everyday PBM Devices

Photobiomodulation (PBM) involves irradiating a living organism at certain energy/power levels to induce biological or biochemical responses. The irradiation may be in the visible spectrum, such as red light, or in the non-visible spectrum, such as infrared (IR). There has been a significant amount of research about the medical benefits of employing PBM therapy to treat physical and psychological symptoms.

Many known devices for administering PBM radiation utilize very high power levels to irradiate the entire body. Due to these operational requirements, effective PBM treatments have typically been limited to specialized medical or therapeutic equipment, which is often bulky, costly, and inconvenient for everyday use. Consequently, these limitations significantly restrict widespread public access to the beneficial effects of PBM therapy.

In previous applications by the Applicant, specifically in PCT applications published as WO 2023/094648 A1 and WO 2024/028203 A1, which are incorporated herein by reference in their entirety, concepts were introduced for integrating PBM into everyday devices. These concepts describe electro-optical arrangements capable of delivering effective PBM radiation within routine contexts such as general lighting systems, smartphones, monitors, laptops, and other electronic consumer products. The disclosed devices operate by emitting pulsed PBM radiation in the visible red to near-infrared spectral ranges (e.g. 610-1400 nm), alongside or integrated within devices that could be used in daily life such as mobile phones, tablets, computer screens, general lighting fixtures, and more.

Such PBM integration leverages natural usage patterns of these devices, allowing users to passively receive beneficial PBM exposure without requiring dedicated treatment arrangement or time. Furthermore, these previously described devices incorporate sensors (e.g., proximity detectors) to regulate the emission characteristics, such as pulse width, frequency, radiation intensity, spatial or temporal radiation patterns, thereby optimizing the energy consumption and/or ensuring proper dosage levels for effective yet safe PBM delivery.

### Time-of Flight (ToF) Sensors

Time-of-flight (ToF) sensors measure distance by determining the time required for emitted radiation, typically infrared (IR) light, to travel from a sensor to a target, reflect from the target, and return to the sensor's photodetector. Two primary methods for measuring this travel time are known: direct time-of-flight (direct ToF) and indirect time-of-flight (indirect ToF). In direct ToF, the sensor emits a very short-duration pulse of IR radiation and directly measures the precise time interval from emission to detection of the reflected IR pulse. Given the known speed of light, this measured time interval can be accurately converted into a distance measurement.

In contrast, indirect ToF, often referred to as continuous-wave (CW) or phase-based ToF, involves emitting continuously modulated radiation, typically with a sinusoidal intensity modulation. The distance is determined based on measuring the phase shift between the emitted radiation and the radiation reflected back from the target. By evaluating this phase shift, the sensor can infer the time-of-flight indirectly, from which the distance can be computed. The indirect time-of-flight (TOF) principle is typically utilized for 3D imaging with a long integration time.

For fast-ranging applications, such as in the PBM applications, the direct ToF is generally more suitable (and less expensive) due to its superior accuracy, especially at greater distances, and its robustness against ambient light interference. Direct ToF methods can better accommodate the precise and dynamic control of PBM radiation in everyday environments, ensuring accurate distance measurements even under conditions of varying ambient IR levels, such as those introduced by other IR sources or direct sunlight.

ToF sensing technologies typically utilize near-infrared (NIR) radiation sources, such as vertical-cavity surface-emitting lasers (VCSELs) or light-emitting diodes (LEDs), commonly centered around wavelengths such as 850 nm or 940 nm. Optical filters are often used to allow detection primarily of the emitted wavelength, thereby reducing interference from ambient or stray radiation. Because of their accuracy, speed, and compactness, ToF sensors have been widely adopted in applications such as facial recognition, presence detection, gesture control, augmented reality (AR), robotics, automotive safety, and various consumer electronics.

In PBM devices, a ToF sensor can be particularly useful for monitoring the user's distance from the device and dynamically adjusting the emitted PBM radiation intensity and exposure duration. Precise distance measurements enable tailored dose delivery, improved user safety, and/or enhanced energy efficiency by ensuring PBM irradiation only occurs when a user is present within an optimal range.

However, integrating ToF sensors within PBM-enabled everyday devices presents significant challenges. Since PBM sources typically utilize IR radiation within spectral bands (for example around 850 nm) overlapping closely with the emission spectrum used by many ToF sensors (often at approximately 850 nm or 940 nm), spectral overlap between the PBM source and the ToF sensor can occur. This spectral overlap can cause the PBM radiation to act as a source of stray or ambient IR light to the ToF sensor's photodetector, thus affecting its ability to accurately measure the distance.

Therefore, there is a need to provide an improved electro-optical arrangement wherein precise ToF measurements can reliably coexist with effective photobiomodulation irradiation, without causing mutual interference.

### SUMMARY OF THE INVENTION

The first aspect of the present invention relates to an electro-optical arrangement according to claim 1.

The electro-optical arrangement (which may comprise one or more devices) according to the present invention utilizes two red or infrared radiation sources (which can optionally be integrated into a single source in some embodiments): a first radiation source operable to emit a first pulsed radiation beam having a peak emission wavelength between 610 and 1400 nm (which can serve as a PBM radiation source, and this pulsed radiation beam can emulate pulsed or continuous wave PBM emissions as described below), and a second radiation source operable to emit a second pulsed radiation beam having a peak emission wavelength between 610 and 1400 nm (which can serve as a ToF emitter; the peak emission wavelength is preferably in a range of 810-940 nm, where typical wavelengths include, but are not limited to, 810 nm, 830 nm, 850 nm, 905 nm, and 940 nm). The second pulsed radiation beam comprises a plurality of pulses emitted with a periodicity defined by a pulse repetition period of up to 20 µs within a recurring ranging period of up to 2000 ms. A receptor is operable to detect a reflected portion of said second pulsed radiation beam from a target and to determine a distance to the target based on said detection during one or more ranging periods. A radiation control unit is operable to adjust at least one emission parameter of the first radiation source (such as radiation intensity, duty cycle, and/or pulse frequency of the first pulsed radiation beam) based on the determined distance. In this way, the PBM radiation can be dynamically adapted according to accurate distance measurements, thereby providing efficient PBM delivery and improved dose control.

A crucial technical consideration is avoiding interference between the two radiation sources. One viable option is to have the radiation control unit operable to alternately activate the first and second radiation sources, such that emission of the first pulsed radiation beam does not temporally overlap with emission of the second pulsed radiation beam. However, this alternating approach may lead to an undesirably low pulse frequency for the first radiation source. Specifically, since ToF sensors typically require a relatively long ranging period (commonly 30-200 ms), alternately activating the first radiation source only after completion of each ranging period (e.g., every 50 ms) may result in a pulse frequency below 20 Hz. As described in the applicant's earlier applications (e.g., WO 2023/094648 A1), it is desirable that the PBM radiation is emitted at pulse frequencies corresponding to one or more of the following frequencies: at least 100 Hz, 30 Hz or an integer multiple thereof, and/or 120 Hz or an integer multiple thereof, thereby avoiding noticeable flicker effects from the perspective of a user or a camera sensor (so-called "image flicker"). In addition, if the peak wavelength of the first pulsed radiation beam is relatively low (e.g. at 850 nm), a pulse frequency above 100 Hz is desirable since such spectrum is somewhat visible to human eyes.

In light of the above considerations, the electro-optical arrangement according to claim 1 implements a synchronized emission scheme, in which the first and second radiation sources are time-aligned in a coordinated manner, rather than simply alternating at the level of entire ranging periods. Specifically, a periodicity of the first pulsed radiation beam, defined as inverse of said pulse frequency (which may correspond to one or more of the following frequencies: at least 100 Hz, 30 Hz or an integer multiple thereof, and 120 Hz or an integer multiple thereof), can be synchronized to the second pulsed radiation beam in at least one of the following manners:
- the periodicity of the first pulsed radiation beam is synchronized to the ranging period such that the first pulsed radiation beam comprises a fixed number of pulses during each ranging period, and
- the periodicity of the first pulsed radiation beam is synchronized to the pulse repetition period such that the periodicity of the first pulsed radiation beam corresponds to one or more pulse repetition periods of the second pulsed radiation beam.

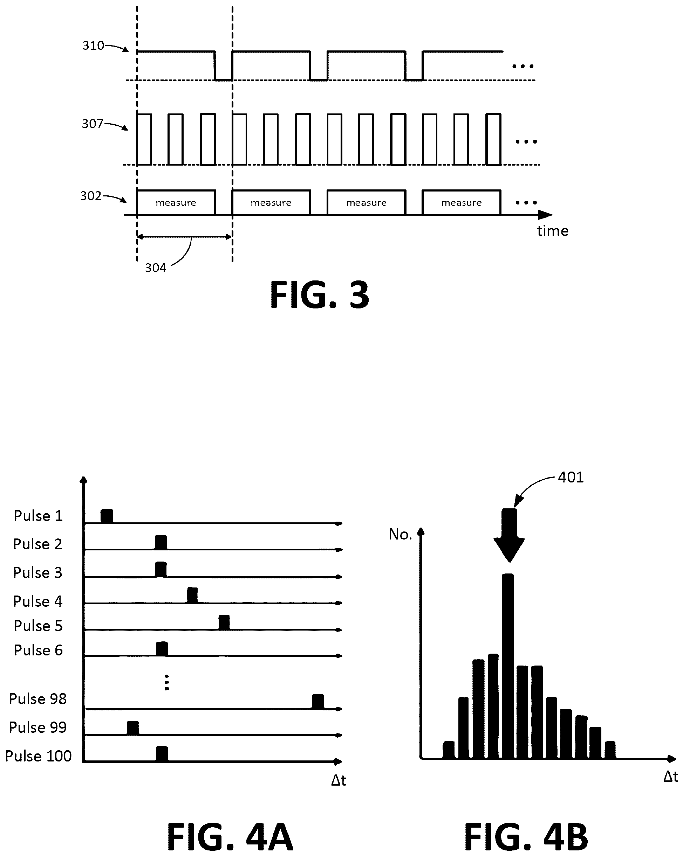

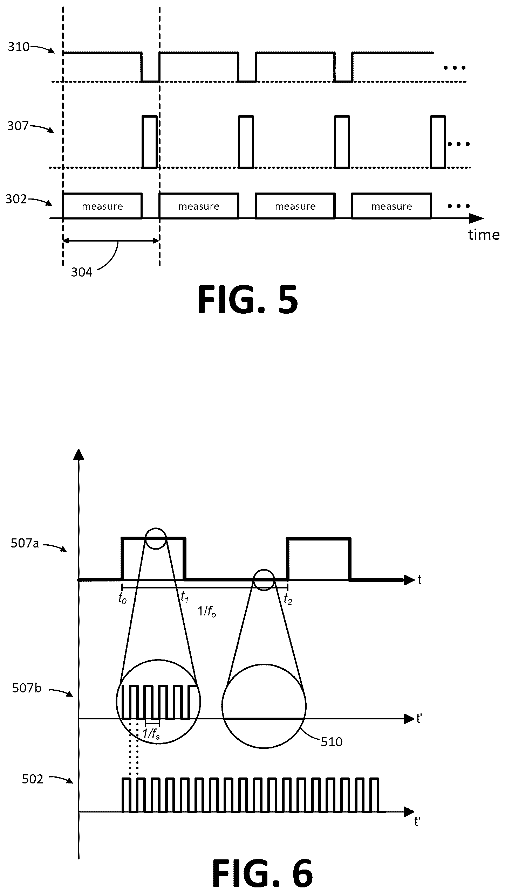

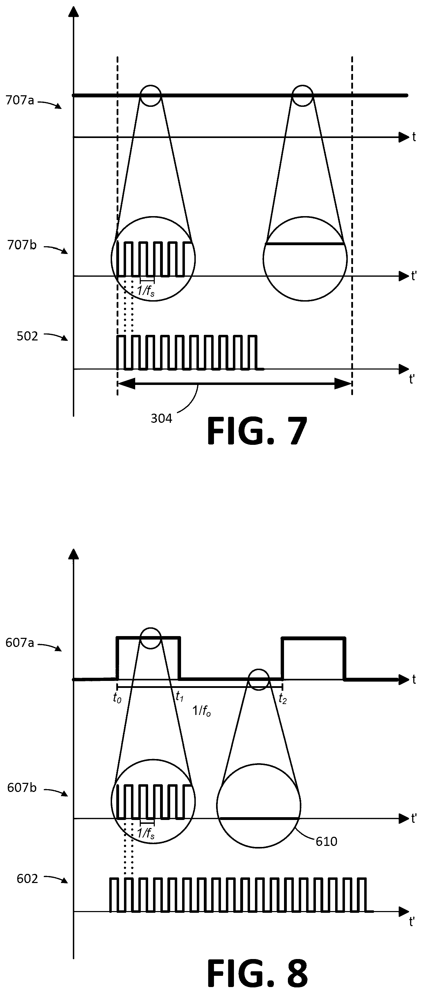

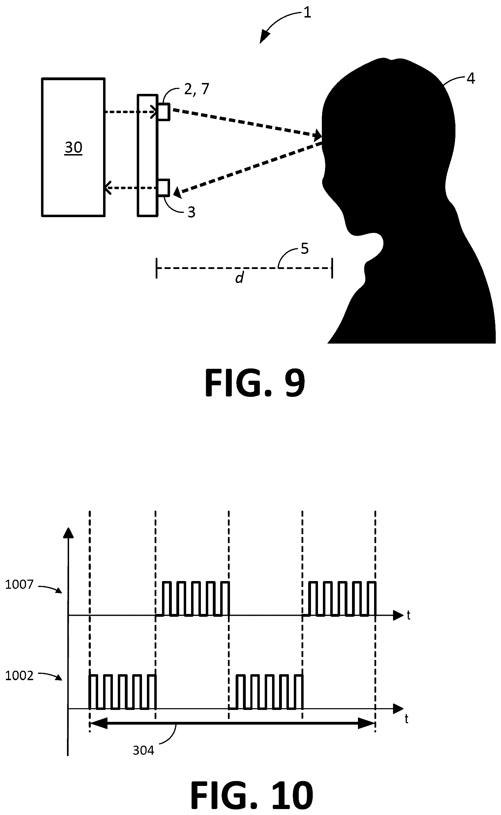

This synchronized emission scheme may be implemented in various ways. In some embodiments, the two radiation sources emit in a non-overlapping sequence within each ranging period (e.g., Figs. 5 and 10) or at the level of each pulse repetition period (e.g., Figs. 6 and 7). In further embodiments, the first and second radiation beams may emit concurrently. Even when temporal overlap occurs, synchronization can ensure that the number of pulses of the first radiation beam within each ranging period is fixed and predictable, such that its influence on the receptor can be identified and filtered using statistical processing (see, e.g., Figs. 3, 4A, and 4B). In yet other embodiments, the first and second radiation beams may emit pulses at precisely the same times (e.g., Fig. 8), or may even be integrated into a single source (e.g., Fig. 9), where synchronizing the emission timing at the pulse level counterintuitively prevents interference. In all such cases, synchronization in the manner set out in claim 1 and the preceding paragraph enables the first radiation source to maintain a pulse frequency sufficient for effective PBM delivery without notable flicker, while minimizing interference with time-of-flight distance measurements performed using the second radiation source.

Several complementary measures can be implemented individually or in combination to further improve the accuracy of the distance measurements. These measures are described in greater detail below.

In an embodiment, the ranging period is less than 60 ms, the periodicity of the first pulsed radiation beam is synchronized to the ranging period, and the receptor comprises a Single Photon Avalanche Diode (SPAD). Using SPAD enables an accurate detection of reflected light at the receptor to the resolution of single photons. In combination with a relatively short ranging period and synchronization with that period, the reflection of the first radiation beam yields a predictable behavior and can be removed through statistics. In other words, this embodiment utilizes low frequency synchronization (at the order below 100 Hz) between the ranging period and the first radiation beam, with predictable interference. In this way, the interference between the two radiation sources can be controlled and an accurate distance measurement can be obtained.

In an embodiment, the first radiation source is driven to emit its pulsed first radiation beam only during a standby interval of each ranging period in which the second radiation source is inactive, such that the first and second pulsed radiation beams do not overlap in time, wherein the second radiation source and the receptor are operable, during each ranging period, to emit and detect the pulsed second radiation beam within a time interval of less than 9 ms, and wherein the distance to the target is determined as a moving average based on measurements performed in a plurality of consecutive ranging periods. This embodiment utilizes low frequency synchronization (at the order around 100 Hz) between a shorter ranging period and the first radiation beam to virtually eliminate interference altogether. Because each ranging period lasts only a few milliseconds, the short measurement intervals significantly reduce the influence of ambient light and interference, while repeated high-speed measurements with a moving average of these rapid measurements can maintain accuracy by smoothing random noise and transient signal fluctuations.

In an embodiment, the electro-optical arrangement further comprises an activation unit operable to generate a periodic activation signal, such as an interrupt, for initiating each ranging period at a predetermined periodicity, wherein, following initiation of each ranging period, the first radiation source is operable to emit a pulse of the first pulsed radiation beam after a predetermined delay. In this way, the two radiation sources are synchronized precisely in time, thereby enhancing accuracy.

In an embodiment, the pulse frequency of the first pulsed radiation beam is at least 50 kHz, such that the periodicity of the first pulsed radiation beam is synchronized to the pulse repetition period. This embodiment utilizes (high frequency) synchronization between the pulses of the TOF emitter and the first radiation beam to reduce interference. The high frequency (micro-)pulses of the first radiation beam can allow the pulses of the first radiation beam to either emit at the same time as the rapid pulses of the second radiation beam, or to emit alternatingly with the pulses of the second radiation beam. In both cases, interference can be reduced and accuracy can be improved.

In an embodiment, the receptor is operable in a gated detection mode synchronized with the emission of the second pulsed radiation beam, such that the receptor is enabled to detect the reflected portion of said second pulsed radiation beam only during a predetermined detection window within each pulse repetition period, and wherein the first radiation source is operable to only emit the first pulsed radiation beam outside the predetermined detection window. In this way, the receptor is gated during the emission of the first radiation beam (PBM radiation source) while it operates when only the second radiation beam (ToF emitter) emits, enabling an accurate distance measurement.

In an embodiment, the first radiation source is operable to emit each pulse of the first pulsed radiation beam only during the emission of the pulses of the second pulsed radiation beam. For example, each pulse of the first pulsed radiation beam may be synchronized with one pulse of the second pulsed radiation beam. Such configuration ensures accurate distance measurement by preventing interference between the two radiation beams. By emitting the two radiation beams in concert, the first radiation beam no longer introduces stray light into the receptor at incorrect times. Instead, both beams arrive simultaneously at the receptor, enabling correct distance determination.

In an embodiment, the first and second radiation sources are integrated, operable to emit a joint radiation beam that acts both as said first and second pulsed radiation beams, to induce photobiomodulation (PBM) at a user while a reflected portion of the joint radiation beam from the user is used by the receptor to determine a distance from the user. This embodiment builds on the previous embodiment to use one radiation source to perform both functions of the two radiation beams, which can be viewed as a perfect synchronization of the two radiation beams, thereby eliminating interference entirely.

In an embodiment, a plurality of (micro-)pulses of the first pulsed radiation beam collectively define a macro-pulse having a macro-pulse repetition frequency and a macro-pulse duty cycle of less than 50%, wherein the macro-pulse repetition frequency corresponds to one or more of the following frequencies: at least 100 Hz, 30 Hz or an integer multiple thereof, and 120 Hz or an integer multiple thereof. This configuration allows a "dual pulse" mode to be defined: the (micro-)pulses are synchronized with the rapid pulses of the second radiation beam, reducing interference and improving distance measurement, while the macro-pulses are set with an appropriate frequency and duty cycle to deliver the desired PBM dose. Together, these enable highly accurate and controllable PBM delivery.

In an alternative embodiment, a plurality of pulses of the first pulsed radiation beam collectively emulate a continuous wave (CW) emission. Despite the emission of the first radiation beam looks like a continuous wave emission from the user's perspective, the (micro-)pulses within this emission can be properly synchronized with the pulses of the second radiation beam in the manners described above, thereby reducing interference and improving accuracy of distance measurement.

In an embodiment, the ranging period is in a range of 300-1000 ms. Synchronizing the pulses of the first and second radiation beams enables more flexibility in setting the duration of the ranging period, and a longer ranging period improves accuracy while an update of distance measurement around once per second (corresponding to a 1000 ms ranging period) is also sufficient for PBM delivery purposes.

In an embodiment, the at least one parameter of the first radiation source includes one or more of: radiation intensity, pulse frequency, and duty cycle. Preferably, adjusting the at least one emission parameter includes adjusting the radiation intensity (including adjusting maximum amplitude of a driving current) while the pulse frequency and duty cycle are kept constant.

In an embodiment, the first radiation source is operable to direct the first pulsed radiation beam towards the target, such that the first pulsed radiation beam has a peak irradiation intensity above 0.1 mW/cm² at a surface of the target. Utilizing the accurate distance measurement of the present invention, the system as a whole can be adapted to deliver sufficient irradiation intensity at all times to induce PBM in the user despite movement of the user.

In an embodiment, the first radiation source is operable to emit the first pulsed radiation beam at a first wavelength, and the second radiation source is operable to emit a pulsed radiation beam at a second, different wavelength, wherein a control unit is arranged to alternately activate the first and second radiation sources in mutually exclusive time windows that repeat within successive ranging periods, the receptor being operable to detect the respective reflected pulses, so that the distance is determined on the basis of both the first and second pulsed radiation beams. In this way, distance data obtained at the two wavelengths can be combined to suppress ambient-light interference and extend the unambiguous measurement range. For example, the second radiation source can operate at a wavelength of 940 nm to act as one Time of Flight (TOF) light source, used alongside the first radiation source at a wavelength of 850 nm, acting both as a PBM source and TOF emitter. This setup provides additional information for post-processing, allowing for more effective suppression of ambient light disturbances.

In an embodiment, the electro-optical arrangement further comprises a calibration unit operable to perform a calibration by measuring a level of ambient radiation detected by the receptor during activation of the first radiation source and to compensate distance measurements based on said calibration. By taking into account the influence of the first pulsed radiation beam during calibration, the accuracy of distance measurement can be further improved.

### BRIEF DESCRIPTION OF THE DRAWINGS

Embodiments will now be described, by way of example only, with reference to the accompanying schematic drawings in which corresponding reference symbols indicate corresponding parts, and in which:
Figs. 1 schematically shows a ToF sensor system that may be used in the embodiments of the present invention.
Fig. 2 schematically shows an embodiment of an electro-optical arrangement 1 according to the present invention.
Fig. 3 schematically shows an embodiment of synchronized emissions of the first and second radiation sources, suitable for use in the electro-optical arrangement 1 of the invention.
Fig. 4 schematically shows an implementation in which the receptor 3 comprises a Single Photon Avalanche Diode (SPAD).
Fig. 5 schematically shows an embodiment in which the emission of the second radiation source (TOF emitter) coincides with the OFF period of the first radiation source while a desired pulse frequency threshold is still achieved.
Fig. 6 schematically shows an embodiment of synchronized emissions of the first and second radiation sources, suitable for use in the electro-optical arrangement 1 of the invention
Fig. 7 schematically shows an embodiment similar to Fig. 6, while a plurality of pulses of the first pulsed radiation beam collectively emulate a continuous wave (CW) emission.
Fig. 8 schematically shows an embodiment of synchronized emissions of the first and second radiation sources, suitable for use in the electro-optical arrangement 1 of the invention.
Fig. 9 schematically shows an embodiment in which the first radiation source 7 and the second radiation source 2 are integrated into a single source.
Fig. 10 schematically illustrates an embodiment in which the first and second radiation sources emit high-frequency pulses at different wavelengths in an alternating manner within each ranging period. This time-division synchronization enables the receptor to perform distance measurements at multiple wavelengths, thereby emulating the principles of multi-wavelength interferometry.

### DESCRIPTION OF ILLUSTRATIVE EMBODIMENTS

The following is a description of certain embodiments of the invention, given by way of example only and with reference to the drawings.

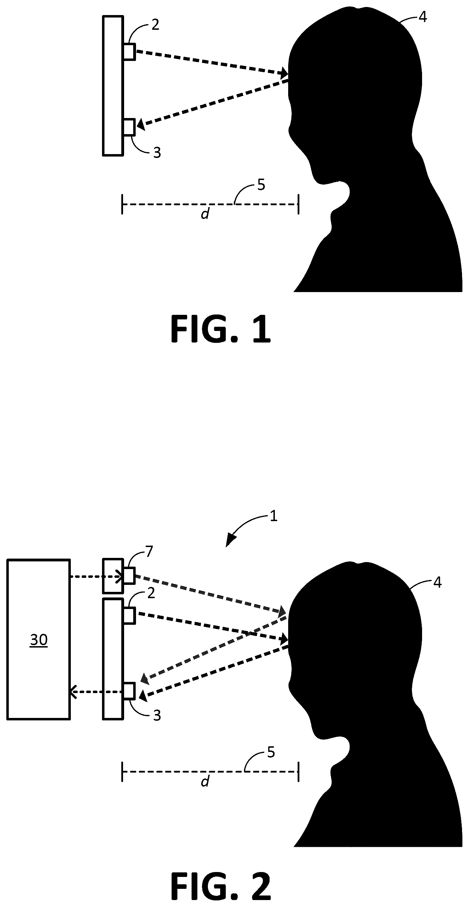

Fig. 1 shows a ToF sensor system having a (second) radiation source 2 (e.g. an emitter) and a receptor 3 (e.g. a sensor), which may be used in the embodiments according to the present invention. In this configuration, the radiation source 2 emits light signals, and the receptor 3 detects light that is reflected from a target 4, such as a user. The system is operable to measure the distance 5 between the device 1 and the target 4 by utilizing the principle of ToF measurement.

In this embodiment, the radiation source 2 is a light source that generates a pulse of light. A pulse of light is generated when photons are emitted substantially simultaneously or in a synchronized manner, resulting in a burst of radiation characterized by specific parameters, including pulse duration, intensity, and wavelength. Upon activation, the radiation source 2 releases a pulse towards the target 4 with a defined duration and wavelength, allowing it to be distinguishable from ambient light and any other sources of radiation in the measurement area.

In a preferred embodiment, the wavelength of the emitted light pulse is within the infrared (IR) spectrum, typically ranging from 700 nm to 1,400 nm. The infrared (IR) spectrum is preferred as the emitted light at these wavelengths is less susceptible to scattering by atmospheric particles, ensuring more reliable measurements. Furthermore, infrared light is generally less affected by visible light from surrounding sources, improving the signal-to-noise ratio in the sensor's detection system. The peak emission wavelength is preferably in a range of 810-940 nm, including 810 nm, 830 nm, 850 nm, 905 nm, and 940 nm.

The pulse travels from the radiation source 2 towards target 4. Upon encountering the target 4, a portion of the emitted light is reflected towards the receptor 3. The receptor 3 is responsible for detecting the reflected pulse that is emitted by the radiation source 2 and may include a photodetector or similar light-sensitive element that is capable of capturing the returning light pulse.

The receptor 3 may include a timing circuit (not shown) that measures the time interval from the moment the light pulse is emitted by the radiation source 2 to the moment it is detected by the receptor 3 after reflection. Specifically, the receptor 3 may be arranged to determine the distance to the target based on a measured time interval between emission of the light pulse and detection of the reflected portion thereof, e.g. using a processor, an ASIC, an FPGA, etc., which calculates the distance to the target 4 based on the speed of light. The distance D can be determined from the equation below: $D = c \times t / 2$

Where c is the speed of light and t is the time interval between emission and detection. The factor of 2 accounts for the round-trip journey of the light pulse, from the radiation source 2 to the target 4 and back to the receptor 3.

Fig. 2 schematically shows an embodiment of an electro-optical arrangement 1 according to the present invention. The electro-optical arrangement is equipped with the ToF sensor system as described in Fig. 1. In addition, it comprises a first radiation source 7 for inducing PBM effect at the user. The components of the electro-optical arrangement 1 may physically be embodied in the same or different devices. The first radiation source 7 may comprise one or more infrared (IR) LEDs.

The first radiation unit may be adapted to emit a first radiation beam having a peak emission wavelength between 610-1400 nm. In an embodiment, the peak emission wavelength in in the (near) infrared ((N)IR) region, such as 700-1400 nm. In an embodiment this range may be 760-1400 nm or 800-1100 nm. Another option is the range 800-870 nm. The peak emission wavelength may also be with the spectrum of red light such as the range 610-700 nm. Recent advances in medical research have demonstrated that irradiating a living organism with radiation comprising the (N)IR spectrum and/or red light at certain energy/power levels may induce beneficial biological or biochemical responses. Such irradiation is often referred to as photobiomodulation (PBM). Available medical research results on the medical benefits of employing PBM therapy to treat physical and psychological symptoms are rapidly increasing. Some wavelengths that have attracted particular attention include 606, 627, 630, 632.8, 640, 660, and 670 nm (in the red region) and 785, 800, 804, 808, 810, 820, 830, 850, 904, 980 and 1060 nm (in the NIR region). Some spectral ranges that have attracted particular attention include 650-680 and 800-870 nm. The radiation unit may be configured to emit radiation in multiple wavelength regimes simultaneously.

As shown in this embodiment, a radiation control unit 30 is operable to adjust at least one emission parameter of the first radiation beam (e.g. max current, pulse frequency, and/or duty cycle) based on the determined distance. It is generally accepted that an irradiation intensity greater than 0.1 mW/cm² at the skin's surface is required to induce a meaningful PBM effect. The radiation control unit 30 may be operable to adjust the driving current of the first radiation source 1 in dependence on the determined distance, such that the first radiation beam has a peak irradiation intensity above 0.1 mW/cm², preferably 1 mW/cm² or more at the target 4, which can efficiently induce PBM effect and makes it possible to obtain the desired dose level (e.g. 0.01-50 J/cm², preferably in the range 0.1-20 J/cm², more preferably in the range 1-10 J/cm², more preferably in the range 4-7 J/cm²) within one or more hours. This allows the electro-optical arrangement 1 to be embodied in one or more everyday devices, such as mobile phones, tablets, laptops, phone or tablet covers, webcam or other accessories connected to a display screen, etc., to deliver the desired dose after the everyday use of such devices.

A portion of the light from the first radiation source can be reflected by the target 4 towards the sensor 3. This reflection can cause the receptor 3 to mistakenly detect the reflected LED light as part of the distance measurement signal, leading to inaccurate readings or false measurements. To address this issue, additional measures are used in the present invention as described below.

### First embodiment

Fig. 3 schematically shows a first embodiment of synchronized emissions of the first and second radiation sources, suitable for use in the electro-optical arrangement 1 of the invention.

This Figure illustrates the concept of periodic ranging periods used by a ToF sensor. As shown in row 302, the second radiation source 2 periodically enters active intervals to perform measurement, followed by a short period in which the second radiation source 2 and receptor 3 may remain inactive or in standby mode. In this application, the term 'ranging period' 304 refers collectively to the active measurement period and the immediately following inactive period. Within each ranging period 304, the second radiation source 2 emits a plurality of short-duration radiation pulses, each pulse being emitted with a short pulse repetition period (e.g. up to 10 µs). For example, the second radiation source may emit light with a duration in a range from 0.2 nanoseconds to 5 nanoseconds and has a repetition rate of 100 kHz to 1 MHz. Reflected portions of these pulses, returned from a target 4, are detected by the receptor 3 during each ranging period to determine the distance to the target.

Row 307 of Fig. 3 represents pulsed emission of the first radiation source 7. In the embodiment shown, the periodicity of the pulses of the first radiation beam is synchronized to the ranging period of the second radiation beam, such that the first radiation source 7 emits a fixed number of pulses (e.g. three as shown in Fig. 3) during each ranging period. Each ranging period is preferably less than 60 ms, more preferably less than 35 ms. In a preferred embodiment, the ranging interval is in a range 10-20 ms, while the standby mode may last for 5 ms (or somewhere between 5-15 ms) between two consecutive ranging intervals.

This arrangement allows the pulses of the first radiation beam to have a sufficient frequency to reduce flicker. For example, if three pulses are emitted every 25 ms (e.g. 20 ms ranging interval plus 5 ms standby), the pulse frequency is 120 Hz, which is not only above 100 Hz, but also a common multiple of the typical frame rates of imaging equipment (e.g. 24, 30 and 60 frames per second), allowing image flicker to be further reduced. At the same time, since the number of pulses of the first radiation beam during each ranging period is fixed, the interference between the first and second radiation beams behaves as outliers during each ranging period, which can be controlled using statistics.

Both the first and second radiation sources may be activated through an interrupt signal 310, so that following initiation of each ranging period, the first radiation source 7 emits a pulse after a predetermined delay (offset), as shown in Fig. 3. An interrupt is a mechanism in microcontroller systems where an event can interrupt the normal flow of execution of the program and immediately trigger a specific action, such as activating a sensor, processing data, or executing a particular function. This event could be triggered by external inputs, such as a button press, a signal from another component or a timer reaching a certain value. In the context of a ToF sensor system, an interrupt can be configured to trigger the second radiation source 2 and receptor 3 to start a measurement process in response to a timer and/or another specific condition or event.

Fig. 4 schematically shows a preferred embodiment, where the receptor 3 comprises a Single Photon Avalanche Diode (SPAD). In this embodiment, the SPAD is configured to detect single-photon events resulting from the reflection of individual light pulses emitted by the second radiation source 2. Fig. 4A illustrates a sequence of 100 emitted pulses of the second radiation beam and their corresponding photon detections, shown as short marks along a horizontal time axis Δt. Due to the emission of the first radiation source 7 and other factors such as ambient noise, limited photon return, and quantum randomness, each detection event occurs with some variation in its measured time-of-flight, leading to a spread of arrival times.

The system records these detection events and accumulates them over multiple pulses to construct a statistical distribution of time-of-flight measurements, as shown in Fig. 4B. The horizontal axis again corresponds to Δt, representing the time difference between pulse emission and photon detection, while the vertical axis indicates the number of detected events per time bin. A histogram is formed from these measurements, and the time bin 401 with the highest number of detections is selected as the most probable return time. This peak time corresponds to the most likely distance to the target 4, as it represents the highest confidence interval based on the accumulated photon returns.

This statistical approach enables high-precision distance determination even in conditions of low photon return, high ambient noise, or when fast ranging periods are used. The use of a SPAD allows single-photon resolution and extremely fine temporal accuracy, while histogram-based processing mitigates the effect of outliers and random noise, ensuring a robust and accurate distance measurement. Such an arrangement is particularly advantageous for use in environments with varying lighting conditions caused by the first radiation source 7.

The accuracy of the distance measurement can be further enhanced by calibrating the ToF system to account for background interference. To that end, the electro-optical arrangement 1 may further comprise a calibration unit (which may be part of the radiation control unit 30 or a different entity), operable to perform a calibration procedure by measuring the level of ambient or stray radiation detected by the receptor 3 during activation of the first radiation source 7. This allows the system to characterize any background IR contributions introduced by the PBM emitter, which may otherwise be misinterpreted as part of the reflected signal from the second radiation source 2. Based on this calibration data, the system can compensate the distance measurements accordingly - for example, by subtracting or discounting baseline IR levels from the receptor's output, or by dynamically adjusting detection thresholds during each ranging period. This calibration process may be performed periodically, during system initialization, or upon detecting changes in ambient conditions, ensuring reliable operation across varying environments and usage scenarios.

### Second embodiment

Fig. 5 shows a second embodiment in which the configuration of the first embodiment is adapted so that each ranging period is less than 9 ms, and the distance to the target is determined as a moving average based on measurements performed in a plurality of consecutive ranging periods. Because each individual ranging period lasts only a few milliseconds, the second radiation source 2 emits and the receptor 3 collects data over very short intervals, thus allowing the first radiation beam to be emitted during the standby period in which no active measurement is performed while still maintaining a pulse frequency above 100 Hz. At the same time, even though each individual measurement may contain fewer photons due to the brief sampling window, repeated measurements in rapid succession ensure a sufficient level of statistical confidence. The moving average computation then effectively aggregates these high-speed measurements, smoothing out random noise and any transient fluctuations in signal strength.

This approach offers both fast response and robust accuracy. By rapidly updating the distance reading multiple times per second, the system can track fast changes in the user's position or any intervening objects. Meanwhile, the moving average preserves the precision and repeatability associated with longer observation periods, without requiring the receptor to remain active for extended durations. Consequently, the described second embodiment achieves both reduced susceptibility to external noise sources and responsive distance sensing for dynamic applications.

### Third embodiment

Fig. 6 schematically shows a third embodiment of synchronized emissions of the first and second radiation sources, suitable for use in the electro-optical arrangement 1 of the invention.

In this embodiment, the periodicity of the first radiation beam is synchronized to the rapid pulses of the second radiation beam using a dual pulse operation. In this configuration, the emission of the first radiation source 7 may be viewed as subjected to two distinct electrical pulses within a specified time interval, rather than a continuous or single pulse. In this mode, the output has two distinct pulses: a low-frequency macro-pulse (shown in row 507a with a time scale t) and a high-frequency micro-pulse (shown in row 507b with a zoomed time scale t'). Each micro-pulse of the first radiation beam is synchronized to the pulses of the second radiation beam, shown in row 502.

The macro-pulses have a macro-pulse repetition frequency (fₒ), corresponding to 1/(t₂ - t₀) in Fig. 6. This frequency corresponds to one or more of the following frequencies: at least 100 Hz, 30 Hz or an integer multiple thereof, and 120 Hz or an integer thereof, to reduce flicker. A duty cycle (corresponding to (t₁ - t₀)/(t₂ - t₀) × 100% in Fig. 5) is preferably less than 50%, but a duty cycle greater than 50% is also possible.

The micro-pulses of the first radiation beam (shown in row 507b) is synchronized with the pulses of the second radiation beam (ToF emission), such that the receptor 3 is gated during the emission of the first radiation source 7 while the second radiation source 2 emits during the non-emission of the first radiation source 7. The pulse frequency of the first radiation beam (fₛ in Fig. 5) is preferably at least 50 kHz to match the frequency of the ToF pulses, which typically ranges from 100 kHz to 1 MHz. This arrangement ensures that the first radiation source 7 is turned OFF while the ToF emitter and receptor are ON, allowing an accurate distance measurement with minimal interference.

During the low-frequency OFF time 510 of the first radiation source 7, the ranging period of the second radiation source 2 may continue, as shown in Fig. 6. This allows for long (e.g. 300-1000 ms) and accurate ranging measurements to be conducted without interference during that period. Synchronizing the pulses of the first and second radiation beams enables more flexibility in setting the duration of the ranging period, and a longer ranging period improves accuracy while an update of distance measurement around once per second (corresponding to a 1000 ms ranging period) is also sufficient for PBM delivery purposes.

When the duty cycle of the first radiation source 7 approaches 100%, the first pulsed radiation beam effectively behaves as a continuous wave (CW) radiation from the perspective of a user, as illustrated in Fig. 7. In this state, the plurality of micro-pulses (shown schematically in row 707b) are spaced so closely that they collectively emulate a continuous and uninterrupted emission (shown schematically in row 707a). From the user's viewpoint, the distinction between discrete pulses disappears, and the radiation appears as a steady output without perceptible flicker or modulation.

When the second radiation source 2 and the receptor 3 are not performing active distance measurements, the first radiation source 7 may operate in one of two modes. It may continue emitting the emulated CW radiation using a dense train of micro-pulses, or it may transition to a genuine continuous wave (CW) transmission (as shown in Fig. 7), depending on the system configuration and efficiency considerations. Both modes enable uninterrupted delivery of PBM radiation without causing interference to the time-of-flight ranging operations.

### Fourth embodiment

Fig. 8 shows a fourth embodiment of synchronized emissions of the first and second radiation sources, suitable for use in the electro-optical arrangement 1 of the invention.

The fourth embodiment is similar to the third embodiment and may include the same measures described in that context. However, in this embodiment, the first radiation source 7 and the second radiation source 2 are activated at the same time so that the first radiation source 7 only emits during the emission of the pulses of the second radiation beam. The first radiation source 7 may emit a pulse during every pulse of the second radiation beam, as shown in Fig. 8, or every several pulses of the second radiation beam. etc.

By turning on and off both radiation sources simultaneously, both radiation beams travel towards the target 4, reflected by the target 4, and arrive at the receptor 3 at the same time. This allows an accurate time measurement for determining the distance without interference between the two radiation beams. In other words, by emitting the two radiation beams in concert, the first radiation beam no longer acts as stray light that enters the receptor 3 at a wrong time. Instead, the reflected portion of the first radiation beam arrives at the receptor 3 at the same time as the second radiation beam does, and the time difference between their emission and their reception correctly represents the distance according to the equation D = c × t / 2.

Fig. 9 schematically shows a variant of the fourth embodiment, in which the first radiation source 7 and the second radiation source 2 are integrated into a single source. Its radiation performs a dual function: on the one hand, it generates an emission with sufficient irradiance at the target (at least 0.1 mW/cm²) to induce PBM; on the other hand, the reflected portion of this radiation beam is received by the receptor 3 and is used to perform the distance measurement. The radiation control unit 30 receives the measurement data as an input, and it sends a signal to the integrated radiation source 2, 7 to adjust one or more emission parameters of this beam. By having the integrated radiation source to perform both functions, the interference between two sources can be entirely eliminated.

### Fifth embodiment

Fig. 10 illustrates a further embodiment in which two radiation sources are driven in a time-division sequence at different wavelengths, thereby realising a form of "multi-wavelength interferometry." In the example shown, the second radiation source 1002 is a ToF emitter centred, for instance, around 940 nm, while the first radiation source 1007 is a high-speed PBM emitter centred around 850 nm. The timing controller allocates one complete ranging period 304 exclusively to the ToF channel: during that window the source 1002 fires a train of nanosecond-wide pulses at a pulse-repetition period of, for example, ≤ 10 µs, and the receptor is gated correspondingly to record the time-of-flight information. Immediately after the ToF block ends, the controller hands the timing slot over to the PBM channel; the source 1007 then delivers a burst of micro-pulses whose aggregate ("macro-pulse") provides the desired PBM dose while also serving as a second, spectrally distinct ToF probe.

The receptor may be a single wide-band SPAD array or two narrow-band detectors that are alternately enabled. Because only one wavelength is active during any given ranging period, the detector never receives overlapping pulses, so crosstalk between the 850 nm and 940 nm channels is inherently avoided. The alternation can occur once per ranging period 304, or multiple times as shown in Fig. 10. By comparing the two distance traces obtained in successive measurements, the processor can: (i) identify and reject wrap-around errors extending the unambiguous range, and (ii) subtract common-mode background counts, since ambient IR normally affects the two wavelengths similarly. In this way the dual-wavelength timing sequence emulates many of the benefits of classical multi-wavelength interferometry.

The scheme also preserves user comfort and therapeutic efficacy. The PBM burst in row 1007 comprises closely spaced high frequency micro-pulses, and the resulting macro-pulse train likewise appears at a frequency well above the human flicker-fusion threshold (e.g. ≥ 100 Hz or an integer multiple of 120 Hz). Consequently, the PBM source can deliver its intended dose without degrading the ToF sensor's duty-cycle or accuracy, and the electro-optical arrangement maintains reliable distance sensing even in bright, variable ambient-light environments.

The descriptions above are intended to be illustrative, not limiting. It will be apparent to the person skilled in the art that alternative and equivalent embodiments of the invention can be conceived and reduced to practice, without departing from the scope of the claims set out below.

## Claims

1. An electro-optical arrangement (1), comprising:
a first radiation source (7) operable to emit a first pulsed radiation beam having a peak emission wavelength between 610 and 1400 nm, wherein the first pulsed radiation beam is emitted at a pulse frequency corresponding to one or more of the following frequencies: at least 100 Hz, 30 Hz or an integer multiple thereof, and 120 Hz or an integer multiple thereof;
a second radiation source (2) operable to emit a second pulsed radiation beam having a peak emission wavelength between 610 and 1400 nm, wherein the second pulsed radiation beam comprises a plurality of pulses emitted with a periodicity defined by a pulse repetition period of up to 20 µs, within a reoccurring ranging period of up to 2000 ms;
a receptor (3) operable to detect a reflected portion of said pulsed second radiation beam from a target, and to determine a distance to the target based on said detection during one or more ranging periods; and
a radiation control unit (30) operable to adjust at least one emission parameter of the first radiation source based on the determined distance,
wherein a periodicity of the first pulsed radiation beam, defined as inverse of said pulse frequency corresponding to one or more of the following frequencies: at least 100 Hz, 30 Hz or an integer multiple thereof, and 120 Hz or an integer multiple thereof, is synchronized to the second pulsed radiation beam in at least one of the following manners:
- the periodicity of the first pulsed radiation beam is synchronized to the ranging period such that the first pulsed radiation beam comprises a fixed number of pulses during each ranging period, and
- the periodicity of the first pulsed radiation beam is synchronized to the pulse repetition period such that the periodicity of the first pulsed radiation beam corresponds to one or more pulse repetition periods of the second pulsed radiation beam.

2. The electro-optical arrangement (1) according to claim 1, wherein the ranging period is less than 60 ms, the periodicity of the first pulsed radiation beam is synchronized to the ranging period, and the receptor (3) comprises a Single Photon Avalanche Diode (SPAD).

3. The electro-optical arrangement (1) according to claim 2, wherein the first radiation source (7) is driven to emit its pulsed first radiation beam only during a standby interval of each ranging period in which the second radiation source (2) is inactive, such that the first and second pulsed radiation beams do not overlap in time, wherein the second radiation source (2) and the receptor (3) are operable, during each ranging period, to emit and detect the pulsed second radiation beam within a time interval of less than 9 ms, and wherein the distance to the target is determined as a moving average based on measurements performed in a plurality of consecutive ranging periods.

4. The electro-optical arrangement (1) according to claim 2 or 3, further comprising an activation unit operable to generate a periodic activation signal, such as an interrupt, for initiating each ranging period at a predetermined periodicity, wherein, following initiation of each ranging period, the first radiation source (7) is operable to emit a pulse of the first pulsed radiation beam after a predetermined delay.

5. The electro-optical arrangement (1) according to claim 1, wherein the pulse frequency of the first pulsed radiation beam is at least 50 kHz, such that the periodicity of the first pulsed radiation beam is synchronized to the pulse repetition period.

6. The electro-optical arrangement (1) according to claim 5, wherein the receptor (3) is operable in a gated detection mode synchronized with the emission of the second pulsed radiation beam, such that the receptor (3) is enabled to detect the reflected portion of said second pulsed radiation beam only during a predetermined detection window within each pulse repetition period, and wherein the first radiation source is operable to only emit the first pulsed radiation beam outside the predetermined detection window.

7. The electro-optical arrangement (1) according to claim 5, wherein the first radiation source (7) is operable to emit each pulse of the first pulsed radiation beam only during the emission of the pulses of the second pulsed radiation beam.

8. The electro-optical arrangement (1) according to claim 7, wherein each pulse of the first pulsed radiation beam is synchronized with one pulse of the second pulsed radiation beam.

9. The electro-optical arrangement (1) according to claim 7 or 8, wherein the first radiation source (7) and the second radiation source (2) are integrated, operable to emit a joint radiation beam that acts both as said first and second pulsed radiation beams, to induce photobiomodulation (PBM) at a user while a reflected portion of the joint radiation beam from the user is used by the receptor (3) to determine a distance from the user.

10. The electro-optical arrangement (1) according to any of claims 5-9, wherein a plurality of pulses of the first pulsed radiation beam collectively define a macro-pulse having a macro-pulse repetition frequency and a macro-pulse duty cycle of less than 50%, wherein the macro-pulse repetition frequency corresponds to one or more of the following frequencies: at least 100 Hz, 30 Hz or an integer multiple thereof, and 120 Hz or an integer multiple thereof.

11. The electro-optical arrangement (1) according to any of claims 5-9, wherein a plurality of pulses of the first pulsed radiation beam collectively emulate a continuous wave (CW) emission.

12. The electro-optical arrangement (1) according to any of claims 5-11, wherein the ranging period is in a range of 300-1000 ms.

13. The electro-optical arrangement (1) according to any of the preceding claims, wherein the at least one parameter of the first radiation source includes one or more of: radiation intensity, pulse frequency, and duty cycle.

14. The electro-optical arrangement (1) according to any of the preceding claims, wherein the first radiation source (7) is operable to direct the first pulsed radiation beam towards the target, such that the first pulsed radiation beam has a peak irradiation intensity above 0.1 mW/cm² at a surface of the target.

15. The electro-optical arrangement (1) according to any of the preceding claims, wherein the first radiation source is operable to emit the first pulsed radiation beam at a first wavelength, and the second radiation source is operable to emit a pulsed radiation beam at a second, different wavelength,
wherein a control unit is arranged to alternately activate the first and second radiation sources in mutually exclusive time windows that repeat within successive ranging periods (304), the receptor being operable to detect the respective reflected pulses, so that the distance is determined on the basis of both the first and second pulsed radiation beams.
